# EUROPEAN PATENT APPLICATION

(11) **EP 4 759 332 A1**
(43) Date of publication of application: **17.06.2026**
(21) Application number: 24851913.4
(22) Date of filing: 08.08.2024
(51) Int. Cl.: A61L 2/03, A61L 2/18, C02F 1/50

(54) **STERILIZATION SYSTEM AND STERILIZATION METHOD**

(30) Priority: 10.08.2023 JP 2023130784
(71) Applicant: National Institute for Materials Science, Tsukuba-shi, Ibaraki 305-0047 (JP)
(72) Inventor: EMRAN, Mohammed Youssef Mahmoud, Tsukuba-shi, Ibaraki 305-0047 (JP); OKAMOTO, Akihiro, Tsukuba-shi, Ibaraki 305-0047 (JP)
(74) Representative: Dehns
(86) International application number: PCT/JP2024/028432
(87) International publication number: WO 2025/033496

(57) **Abstract**

A sterilization system for sterilizing a biofilm formed on an object, comprising:
a sterilization composition containing an organic compound having a standard redox potential (pH 7) of -0.7 V to -0.2 V and water;
a sterilization composition supply part that holds the sterilization composition;
a nozzle that supplies the sterilization composition held in the sterilization composition supply part to the biofilm;
an electrode provided in the nozzle so as to be in contact with the sterilization composition; and
a control part connected to the electrode and configured to apply to the electrode a potential exceeding the lower limit of a potential window and being -0.4 V or lower with reference to a silver/silver chloride electrode.

## Description

### [Technical Field]

The present invention relates to a sterilization system and a sterilization method.

### [Background Art]

Currently, infection control in medical settings is a global issue due to biofilms formed by bacterial communities. As a method for sterilizing bacteria, chemical methods using antibiotics and the like are widely known. However, antibiotics are regarded as problematic because the inappropriate use thereof leads to the emergence of drug-resistant bacteria.

Therefore, sterilization techniques other than chemical methods have also been studied over many years. For example, Non-Patent Literature 1 to 4 report a method of removing biofilms formed on the surface of a conductor such as stainless steel or titanium by applying a high potential (for example, about 7 V), which causes H₂ generation and negative-negative surface repulsion.

### [Prior Art Literature]

### [Non-Patent Literature]

[Non-Patent Literature 1] Current Opinion in Solid State and Materials Science, Vol. 25, Issue 4, August 2021, 100926.
[Non-Patent Literature 2] Applied Sciences, 2022, Vol. 12, Issue 13, 6320.
[Non-Patent Literature 3] Bioelectrochemistry 121 (2018) p.84-94.
[Non-Patent Literature 4] Colloids and Surfaces B: Biointerfaces, Vol. 117, 1, May 2014, p.152-157.

However, the techniques disclosed in Non-Patent Literature 1 to 4, while not causing the problem of drug-resistant bacteria, had to be carried out under severe conditions in which electrolysis of solvents and the like occurs. Sterilization under such severe conditions requires a large amount of energy consumption, resulting in high cost, and there is also a risk that active chemical species harmful to the human body may be generated by side reactions.

### [Problem to be Solved by the Invention]

Accordingly, it is an object of the present invention to provide a sterilization system and a sterilization method for sterilizing biofilms, which can be carried out more efficiently under milder conditions.

### [Means for Solving the Problem]

As a result of intensive studies conducted to achieve the above object, the inventors have found that the above object can be achieved by the following embodiment.

[1] A sterilization system for sterilizing a biofilm formed on an object, comprising:
   a sterilization composition containing an organic compound having a standard redox potential (pH 7) of -0.7 V to -0.2 V and water;
   a sterilization composition supply part that holds the sterilization composition;
   a nozzle that supplies the sterilization composition held in the sterilization composition supply part to the biofilm;
   an electrode provided in the nozzle so as to be in contact with the sterilization composition; and
   a control part connected to the electrode and configured to apply to the electrode a potential exceeding the lower limit of a potential window and being -0.4 V or lower with reference to a silver/silver chloride electrode.
[2] The sterilization system according to [1], wherein the electrode is constituted by at least a part of the nozzle.
[3] The sterilization system according to [1], wherein the electrode is constituted by the entire nozzle.
[4] The sterilization system according to any one of [1] to [3], wherein the electrode has a ring shape.
[5] The sterilization system according to any one of [1] to [4], wherein the electrode is a working electrode and further comprises a counter electrode and a reference electrode that are provided so as to be in contact with the sterilization composition.
[6] The sterilization system according to [5], wherein the counter electrode and the reference electrode are provided in the sterilization composition supply part.
[7] The sterilization system according to any one of [1] to [6], wherein the organic compound contained in the sterilization composition is methyl viologen.
[8] The sterilization system according to any one of [1] to [7], wherein the sterilization composition further contains cations.
[9] The sterilization system according to any one of [1] to [8], wherein the sterilization composition further contains silver ions.
[10] The sterilization system according to any one of [1] to [9], wherein the object is a medical instrument.
[11] The sterilization system according to any one of [1] to [10], wherein the object is a tubular body and the biofilm is formed on an inner surface of the tubular body.
[12] The sterilization system according to any one of [1] to [11], wherein the object is an insertion tube of an endoscope.
[13] The sterilization system according to any one of [1] to [12], wherein the control part applies a potential of -1.2 V to -0.8 V to the electrode.
[14] A method for sterilizing a biofilm formed on an object using the sterilization system according to any one of [1] to [13], comprising:
   bringing the sterilization composition into contact with the electrode to which a potential exceeding the lower limit of a potential window and being -0.4 V or lower with reference to a silver/silver chloride electrode is applied; and
   bringing the sterilization composition that has contacted the electrode into contact with the biofilm.

### [Effect of the Invention]

The present invention provides a sterilization system and a sterilization method for sterilizing biofilms, which can be carried out efficiently under mild conditions.

### [Brief Description of the Drawings]

[Fig. 1] A schematic diagram of the sterilization system according to the present embodiment.
[Fig. 2] A schematic diagram illustrating a presumed sterilization mechanism in the sterilization system according to the present embodiment.
[Fig. 3] A schematic diagram illustrating a presumed sterilization mechanism in the sterilization system according to the present embodiment, showing an enlarged view in the vicinity of bacteria.
[Fig. 4] A flowchart illustrating the sterilization method according to the present embodiment.
[Fig. 5] A diagram illustrating the PTFE tube (an object on which a biofilm has formed) used in the examples.
[Fig. 6] A diagram showing evaluation results of sterilization effect (number of colonies) in the examples.
[Fig. 7] A diagram showing evaluation results of sterilization effect (amount of bacterial reduction) in the examples.
[Fig. 8] A diagram showing evaluation results of sterilization effect (number of colonies) in the examples.
[Fig. 9] A diagram showing evaluation results of sterilization effect (amount of bacterial reduction) in the examples.
[Fig. 10] A diagram showing current density of the electrode in the examples.

### [Mode for Carrying Out the Invention]

The description of the constituent elements given below may be based on representative embodiments of the present invention, but the present invention is not limited to such embodiments.
In this specification, a numerical range expressed by "~" means a range including the numerical values described before and after "~" as the lower limit value and the upper limit value, respectively.

### [Sterilization System]

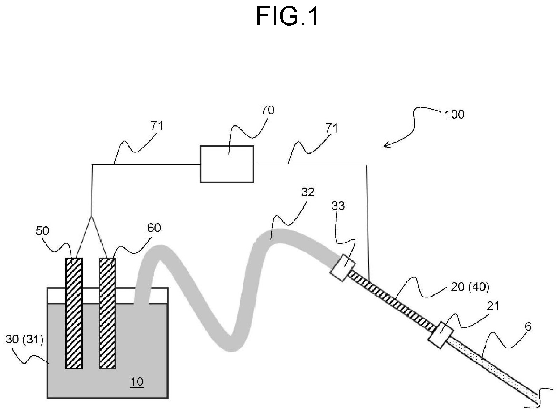

Fig. 1 is a schematic diagram of a sterilization system 100 according to the present embodiment. The sterilization system 100 of this embodiment is a system for sterilizing a biofilm formed on an object (sterilization target) 6. As shown in Fig. 1, the sterilization system 100 of this embodiment comprises, for example, a sterilization composition 10 for sterilizing a biofilm, a nozzle 20 for supplying the sterilization composition 10 to the biofilm, a sterilization composition supply unit 30 for holding the sterilization composition 10, an electrode 40 provided in the nozzle 20, and a control unit 70 connected to the electrode 40 and configured to apply a potential exceeding the lower limit value of the potential window, at -0.4 V or less with reference to a silver/silver chloride electrode, to the electrode 40.

The sterilization composition 10 is a composition for sterilizing a biofilm. The sterilization composition 10 contains an organic compound having a standard redox potential (pH 7) of -0.7 V to -0.2 V (hereinafter appropriately referred to as "specific organic compound") and water. Details of the sterilization composition 10 will be described later.

The sterilization composition supply unit 30 comprises a container 31 for holding the sterilization composition 10. The sterilization composition 10 is held (stored) in the container 31. The sterilization composition 10 is supplied from the container 31 to the biofilm formed on the object 6 via the nozzle 20. Fig. 1 illustrates an embodiment in which the object 6 is connected to the nozzle 20.

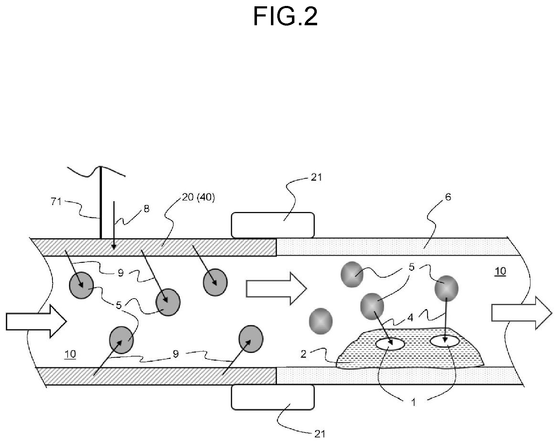

Fig. 2 is a cross-sectional view focusing on a portion where the nozzle 20 and the object 6 are connected according to this embodiment. As shown in Fig. 2, the nozzle 20 is a tubular structure for supplying the sterilization composition 10 to the biofilm 2, and the sterilization composition 10 passes through its interior. The sterilization composition 10 that has passed through the interior of the nozzle 20 is discharged from the nozzle 20 and comes into contact with the biofilm 2. The nozzle 20 can also be referred to as an element for bringing the sterilization composition 10 into contact with the biofilm 2.

In this embodiment, an example is illustrated in which the object 6 is a tubular body and a biofilm 2 is formed on the inner surface of the object 6. The tubular object 6 may be, for example, an insertion tube of an endoscope. Figs. 1 and 2 illustrate an embodiment in which the nozzle 20 also functions as the electrode 40.

Surprisingly, the inventors found that by applying a potential to the electrode 40 and bringing the sterilization composition 10 that has passed through the nozzle 20 provided with the electrode 40 into contact with the biofilm 2 formed on the object 6, the activity of bacteria (bacterial cells) 1 in the biofilm 2 can be reduced, and ultimately sterilization can be achieved, thereby leading to the present invention. Moreover, the negative potential applied to the electrode 40 is sufficient if it is a weak potential within the range of the potential window. This makes it possible to perform sterilization under mild conditions without causing electrolysis of the solvent (water).

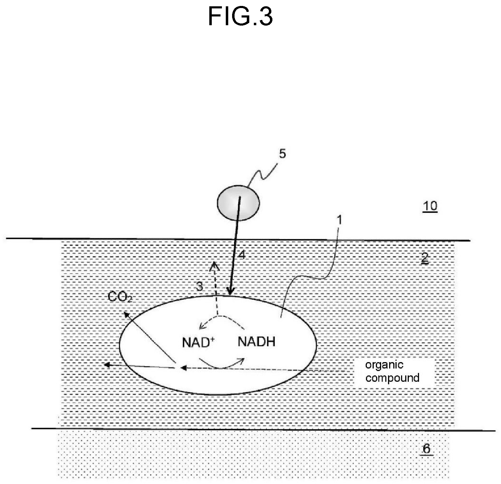

The mechanism of the present invention is presumed to be as follows. Fig. 3 is a schematic diagram illustrating a presumed mechanism in the sterilization method using the sterilization system 100. As shown in Fig. 3, in the process in which the bacterium 1 decomposes an organic compound to obtain energy (particularly under anaerobic conditions), NADH (reduced nicotinamide adenine dinucleotide) is generated from NAD⁺ (oxidized nicotinamide adenine dinucleotide) used in the decomposition of the organic compound. NADH releases an electron (arrow 3) via the electron transport system, returns to NAD⁺, and is used again for energy acquisition.

Fig. 2 schematically shows the potential applied to the electrode 40 and the movement of electrons. As shown in Fig. 2, when a negative potential is applied to the electrode 40 provided in the nozzle 20 (arrow 8), the specific organic compound 5 receives electrons from the electrode 40 (nozzle 20) (arrow 9), passes through the inside of the nozzle 20 in a state where its reducing ability has been enhanced, and comes into contact with or approaches the biofilm 2. Then, as shown in Fig. 3, when the specific organic compound 5 with enhanced reducing ability approaches or comes into contact, the release of electrons (arrow 3) from the bacterium 1 is restricted. In particular, by applying a potential of -0.4 V or less with reference to a silver/silver chloride electrode, electrons move from the specific organic compound 5 to the bacterium 1 (arrow 4), thereby inhibiting energy acquisition in the cell, further lowering its activity, and ultimately killing (sterilizing) the bacterium 1. In this way, the specific organic compound 5 is presumed to act as an electron mediator to facilitate electron transfer from the electrode 40 to the bacterium 1. Therefore, even if the electrode 40 and the bacterium 1 are not in contact, electron transfer occurs from the electrode 40 to the bacterium 1 via the specific organic compound 5, making efficient sterilization possible even with a weak applied potential. This principle, which thermodynamically suppresses bacterial metabolism, is an innovative one that can be expected to have continuous effects on drug-resistant bacteria residing in the biofilm 2. It should be noted that the mechanism described above is presumed, and does not affect the scope of the present invention.

Details of each embodiment of the sterilization system 100 of the present embodiment will be described below. In this specification, the term "sterilization" not only refers to exterminating all bacteria in the biofilm but also includes killing a portion of the bacteria in the biofilm (that is, reducing the number of bacteria in the biofilm).

As described above, the sterilization composition 10 contains the specific organic compound 5 having a standard redox potential (pH 7) of -0.7 V to -0.2 V, and water.

The standard redox potential (pH 7) of the specific organic compound 5 is not particularly limited as long as it falls within the range of -0.7 V to -0.2 V, but from the viewpoint of obtaining a higher sterilization effect, -0.6 V to -0.3 V or -0.6 V to -0.4 V is preferable.

The specific organic compound 5 is not particularly limited as long as it is an organic compound whose standard redox potential falls within the above range. For example, the specific organic compound 5 is preferably a compound containing a nitrogen-containing aromatic heterocycle, and more preferably a compound having a bipyridine structure. Specific examples of such compounds include those listed in the table below and derivatives thereof. Among these, from the viewpoint of obtaining a higher sterilization effect, methyl viologen and its derivatives are preferable, with methyl viologen being more preferable. The specific organic compound 5 may be composed of a single compound or of two or more compounds.

**[Table 1]**

| Specific Organic Compound | Standard Redox Potential (pH7) [V] |
|---|---|
| Safranin T | -0.289 |
| Indulin Scarlet (CAS No. 2611-49-6) | -0.299 |
| Natural Red (CAS No. 553-24-2) | -0.325 |
| 1,1'-dibenzyl-4,4'-bipyridinium dichloride (Benzyl viologen) | -0.358 |
| 1,1'-Ethylene-2,2'-bipyridylium ion (Diquation) | -0.361 |
| 1,1'-Bis(2-hydroxyethyl)-4,4'-bipyridinium | -0.408 |
| 1,1'-dimethyl-4,4'-bipyridinium dichloride (Methyl viologen) | -0.440 |
| 1,1'-diheptyl-4,4'-bipyridinium | -0.480 |
| 1,1'-Diethyl-[4,4'-bipyridine]-1,1'-diium bromide (Ethylviologen Dibromide) | -0.495 |
| 1,1'-diisopropyl-4,4'-bipyridinium dichloride | -0.495 |
| 1,1'-trimethylene-2,2'-bipyridyl | -0.540 |
| 5,5'-dimethyl-2,2'-bipyridyl | -0.664 |
| Carminic acid | - |
| Phenazine-1,6-dicarboxylic acid | - |
| Tetrazine | - |
| Indigo carmine | - |

The content of the specific organic compound 5 in the sterilization composition 10 is not particularly limited, but from the viewpoint of obtaining a higher sterilization effect, it is, for example, 1 µM (M = mol/L) to 1000 µM, or 10 µM to 600 µM.

The water contained in the sterilization composition 10 is not particularly limited and may be purified water, distilled water, ion-exchanged water, or the like.

The sterilization composition 10 may further contain cations. By using the specific organic compound 5 in combination with the cations, a synergistic effect can be produced, thereby further enhancing the sterilization effect. Although the mechanism is not clear, it is presumed as follows. When a negative potential is applied to the electrode 40 in the sterilization composition 10 containing the specific organic compound 5, electron transfer to the bacterium 1 is promoted (arrow 4 in Fig. 3). At this time, if cations are contained in the sterilization composition 10, the cations flow into the bacterium (bacterial cell) 1. As a result, osmotic pressure abnormalities occur inside the bacterium (bacterial cell) 1, reducing the bacterial activity and ultimately leading to sterilization. In addition, when the cations have the property of damaging DNA or enzymes within the bacterium 1, the sterilization effect can be further enhanced. It should be noted that the mechanism described above is presumed, and does not affect the scope of the present invention.

The cations are not particularly limited and may include, for example, cations containing metals such as metal ions or complex ions, and organic cations generated from drugs or the like. Examples of metals contained in the metal ions and metal-containing cations include silver (Ag), copper (Cu), cobalt (Co), aluminum (Al), nickel (Ni), zinc (Zn), molybdenum (Mo), vanadium (V), zirconium (Zr), tungsten (W), palladium (Pd), and platinum (Pt). From the viewpoint of further enhancing the sterilization effect, silver ions (Ag⁺) and copper ions (Cu⁺, Cu²⁺) are preferable, and silver ions (Ag⁺) are more preferable. The cations may also be cations derived from so-called physiological electrolytes, such as sodium ions (Na⁺), potassium ions (K⁺), calcium ions (Ca²⁺), and magnesium ions (Mg²⁺). As the cations, one kind may be used, or two or more kinds may be used.

The sterilization composition 10 may further contain anions serving as counter ions to the cations. The anions are not particularly limited, and examples include halide ions such as fluoride, chloride, bromide, and iodide ions. The cations may be ions derived from salts composed of cations and anions. Preferred salts include halide metals such as silver chloride and copper chloride. By dispersing or dissolving (including partial dissolution) such salts in the sterilization composition 10, cations can be introduced into the sterilization composition 10.

The concentration of the cations in the sterilization composition 10 is not particularly limited and can be appropriately adjusted within a range in which the effects of the present embodiment are exhibited. From the viewpoint of obtaining a higher sterilization effect, for example, the concentration of the cations in the sterilization composition may be 1 µM to 1000 µM, or 10 µM to 200 µM.

The sterilization composition 10 may be composed only of the specific organic compound 5 and water, or may contain, within a range in which the effects of the present embodiment are exhibited, other components in addition to the specific organic compound 5 and water. Examples of such other components include, in addition to the above-described cations, electron mediators other than the specific organic compound 5, electron donor compounds, buffering agents, and gelling agents. The electron donor compound (electron donor) is a compound used in bacterial metabolism, and is not particularly limited, but examples include organic compounds such as amino acids, sugars, and organic acids. The buffering agent is not particularly limited, but examples include borate, bicarbonate, Tris-HCl, citrate, phosphate, succinate, and acetate. The gelling agent may be, for example, agar, gelatin, or agarose, and agar is preferable.

General culture media (liquid media or solid media), buffer solutions, and physiological saline contain water and the above-described other components. Therefore, the sterilization composition 10 of the present embodiment may be a mixture of the specific organic compound 5 and a general culture medium or the like.

The sterilization composition 10 can be prepared by uniformly mixing the specific organic compound 5, water, and, if necessary, other components by a conventional method. Alternatively, the sterilization composition 10 may be prepared by adding a predetermined amount of the specific organic compound 5 to a general culture medium, buffer solution, or physiological saline. Commercially available media, buffer solutions, and physiological saline may be used.

As described above, when the object 6 to be sterilized is a tubular body and the biofilm 2 formed on the inner surface of the tubular body is to be sterilized, as shown in Figs. 1 and 2, the nozzle 20 and the object (tubular body) 6 may be connected by means of a joint 21 (for example, a silicone tube), and the sterilization composition 10 may be allowed to flow from the nozzle 20 into the interior of the object (tubular body) 6. The nozzle 20 and the object 6 are connected to each other by means of the joint 21 so that the inside (flow path) of the nozzle 20 is connected to the inside (flow path) of the object 6. However, the shape of the object 6 is not limited to a tubular body. For example, when the object 6 is a tooth of a human or another organism and a biofilm formed inside a pore formed in the tooth is to be sterilized, the nozzle 20 may be inserted into the pore, and the sterilization composition 10 may be allowed to flow into the pore.

The material of the nozzle 20 is not particularly limited, and a metal material or various plastic materials can be used. As the metal material, the same metal materials as those used for the electrode 40 described later can be used, and the preferred embodiments are also the same. When the nozzle 20 is made of a metal material, it is preferable because the whole or part of the nozzle 20 can function as the electrode 40. Examples of various plastics include polyvinyl chloride, polycarbonate, ABS resin, polypropylene, polyethylene, polystyrene, fluororesin, polyethylene terephthalate, methyl methacrylate, polyamide, polyethersulfone, polysulfone, polyurethane, ethylene-vinyl acetate copolymer resin, silicone resin, thermoplastic elastomer, and liquid silicone rubber (LSR). The nozzle 20 may be composed of a single material or may be a composite composed of a plurality of materials.

The size (outer diameter) of the nozzle 20 is not particularly limited and can be appropriately designed according to the object 6 to be sterilized. For example, when the object 6 is an insertion tube of an endoscope, the size (outer diameter) of the nozzle 20 is preferably not greatly different from that of the insertion tube, and may be, for example, about 1 mm to 100 mm.

The electrode 40 is provided in the nozzle 20 so as to be in contact with the sterilization composition 10 passing through the nozzle 20. By providing the electrode 40 in the nozzle 20, the electrode 40 can be brought closer to the biofilm 2, which is the sterilization target. This makes it possible to send a larger amount of the specific organic compound 5, whose reducing ability has been enhanced by receiving electrons from the electrode 40, toward the biofilm 2, thereby further enhancing the sterilization effect.

As illustrated in Figs. 1 and 2, the electrode 40 may be constituted by a part or the whole of the nozzle 20. When a part or the whole of the nozzle 20 is used as the electrode 40, it becomes easier to enlarge the electrode 40 and to increase the contact area between the electrode 40 and the sterilization composition 10. As a result, the amount (concentration) of the specific organic compound 5 whose reducing ability has been enhanced can be increased, thereby further enhancing the sterilization effect.

The electrode 40 may also be provided in the nozzle 20 as a separate member from the nozzle 20. When the electrode 40 is provided as a separate member from the nozzle 20, an embodiment may be adopted in which, for example, a tubular electrode 40 is provided at the tip of the nozzle 20, an embodiment in which the electrode 40 is provided inside the nozzle 20, or an embodiment in which a wire-shaped electrode 40 extends from the inside of the tubular nozzle 20. The electrode 40 may be ring-shaped so that the sterilization composition 10 flows through its interior. The size (outer diameter) and the length (length along the flow direction of the sterilization composition 10) of the electrode 40 in this case are not particularly limited and can be appropriately designed according to the object 6 to be sterilized or the like. The size (outer diameter) of the electrode 40 may be approximately the same as that of the nozzle 20 described above. The length of the electrode 40 may be, for example, about 0.1 cm to 30 cm, or 0.5 cm to 10 cm.

The material constituting the electrode 40 is not particularly limited as long as it is a conductor capable of applying a potential. Examples of the material include metal materials such as silver, copper, aluminum, nickel, iron, and alloys containing these metals (for example, stainless steel (SUS)), and carbon materials such as amorphous carbon, graphite, and carbon nanotubes.

In the present embodiment, the electrode 40 serves as a working electrode used in a three-electrode method, and two additional electrodes (a counter electrode 50 and a reference electrode 60) may also be provided. By using the three-electrode method, the potential applied to the electrode (working electrode) 40 can be controlled more accurately and easily. The counter electrode 50 and the reference electrode 60 are not particularly limited in their placement as long as they are in contact with the sterilization composition 10, but it is preferable to provide them in the sterilization composition supply part 30. By providing the counter electrode 50 and the reference electrode 60 in the sterilization composition supply part 30 and providing only the electrode (working electrode) 40 in the nozzle 20, the nozzle 20 can be made smaller compared with an embodiment in which the counter electrode 50 and the reference electrode 60 are also provided in the nozzle 20 in addition to the electrode 40. However, an embodiment in which the counter electrode 50 and the reference electrode 60 are also provided in the nozzle 20 in addition to the electrode 40 may also be adopted. The materials of the counter electrode 50 and the reference electrode 60 are not particularly limited, and known counter electrodes and reference electrodes used for electrochemical measurements or the like can be used. The reference electrode 60 is preferably a silver/silver chloride electrode.

The current density of the current involved in the sterilization reaction flowing through the electrode 40 during sterilization is preferably -10 µA/cm² to -0.01 µA/cm², and more preferably -10 µA/cm² to -0.1 µA/cm². By setting the current density at the electrode 40 within the above range, sterilization can be performed with a small current value, providing the advantage of suppressing side reactions on the electrode surface. In addition, by setting the current density at the electrode 40 within the above range, damage to the object 6 during cleaning can be reduced, and even when used in the body, the influence on biological tissues can be minimized.

The current flowing through the electrode 40 during sterilization includes, in addition to the current involved in the sterilization reaction, a system-specific background current that is unrelated to the sterilization reaction. The "current density of the current involved in the sterilization reaction" refers to the value obtained by subtracting the system-specific background current from the current measured for the electrode 40, and dividing the result by the contact area between the electrode 40 and the sterilization composition 10. The method for calculating the "current density of the current involved in the sterilization reaction" is not particularly limited, but may be calculated, for example, by the method described in the Examples.

As illustrated in Fig. 1, the container 31 of the sterilization composition supply part 30 is connected to the nozzle 20 via, for example, a tube 32 and a joint 33, and is provided so as to supply the sterilization composition 10 to the nozzle 20. However, the container 31 and the nozzle 20 may be directly connected. The materials of the container 31, the tube 32, and the joint 33 are not particularly limited and may be appropriately selected from general materials. In addition, as described above, the sterilization composition supply part 30 may further include the counter electrode 50 and the reference electrode 60 as necessary.

The supply of the sterilization composition 10 from the sterilization composition supply part 30 to the nozzle 20 can be performed by, for example, a liquid feeding mechanism (not shown). The sterilization system 100 of the present embodiment may or may not include the liquid feeding mechanism as one of its components. If not included, a liquid feeding mechanism (liquid feeding device) separate from the sterilization system may be used. Examples of such liquid feeding mechanisms include general liquid feeding pumps such as tube-type roller pumps and syringe pumps.

The sterilization composition 10 supplied from the sterilization composition supply part 30 to the nozzle 20 further passes through the nozzle 20, flows to the object 6, and comes into contact with the biofilm 2 to sterilize it. The sterilization composition 10 that has come into contact with the object 6 may subsequently be discharged (drained) as is, or may be returned again to the sterilization composition supply part 30. For example, when the object 6 is a tubular body such as an insertion tube of an endoscope, the outlet (opening on the side opposite to the nozzle 20) of the object 6 (tubular body) and the sterilization composition supply part 30 may be directly connected, or connected using a tube or the like. In this way, the sterilization composition 10 can circulate between the sterilization composition supply part 30, the nozzle 20 (electrode 40), and the object 6 (tubular body), thereby enabling more efficient sterilization of the biofilm.

The control part 70 is connected to the electrode 40 via, for example, a wire 71, and applies a predetermined potential to the electrode 40. Specifically, the control part 70 applies to the electrode 40 a potential that exceeds the lower limit value of the potential window and is -0.4 V or lower with reference to a silver/silver chloride electrode. Any known device capable of applying a potential can be used as the control part 70. When the three-electrode method is used, the control part 70 may be a potentiostat that is connected to the three electrodes (the electrode 40, the counter electrode 50, and the reference electrode 60) via the wire 71 and controls these electrodes.

The inventors have found that, regardless of the type of bacterium, by using the sterilization composition of the present embodiment and applying a weak potential of -0.4 V or lower, electron transfer (arrow 4 in Figs. 2 and 3) from the electrode 40 to the bacterium 1 via the specific organic compound 5 is promoted, thereby inhibiting the energy acquisition of the cell and ultimately achieving sterilization. From the viewpoint of obtaining a higher sterilization effect, it is preferable to apply to the electrode 40 a potential of -0.6 V or lower, -0.8 V or lower, or -0.9 V or lower. In addition, the lower limit value of the negative potential applied to the electrode 40 is not particularly limited as long as it exceeds the lower limit value of the potential window. The lower limit value of the potential window is determined depending on the components, pH, and the material of the electrode 40 of the sterilization composition 10, and is apparent to those skilled in the art. When the applied potential exceeds the lower limit value of the potential window, sterilization can be performed under mild conditions without causing electrolysis of the solvent (water). The sterilization method of the present embodiment is low-cost because it can reduce energy consumption, and can also suppress side reactions that may generate reactive chemical species having adverse effects on the human body. Furthermore, when a high potential is applied and electrolysis of water occurs, reactions such as hydrogen evolution are prioritized, which may suppress electron transfer (arrow 4 in Figs. 2 and 3) from the sterilization composition 10 to the bacterium 1. In the present embodiment, since the applied potential is weak, generation of hydrogen or the like does not occur, and sterilization can be achieved by efficiently suppressing the metabolism of the bacterium 1. The lower limit value of the potential is preferably -1.4 V or higher, -1.2 V or higher, or -1.1 V or higher with reference to a silver/silver chloride electrode.

### [Sterilization Target]

The object 6 to be sterilized is not particularly limited, but examples include medical instruments. Examples of medical instruments include, for example, instruments that are used by penetrating or directly contacting the mucous membrane of a living body (for example, forceps or endoscopes), and instruments that are used by being implanted in a living body (for example, various implants such as dental implants). In some cases, biofilms may form in fine portions of such medical instruments where mechanical cleaning is difficult. Even bacteria contained in such biofilms can be killed (sterilized) by the sterilization method of the present embodiment, and thus prevention of bacterial infection can be expected. In particular, the sterilization system 100 of the present embodiment can allow the sterilization composition 10 to flow from the tip of the nozzle 20 into the interior of the tubular object 6, thereby efficiently sterilizing a biofilm formed on the inner surface (inner wall surface) of the tubular body (for example, an insertion tube of an endoscope). The object 6 may also be, for example, a tooth of a human or another organism. When sterilizing a biofilm formed inside a pore formed in a tooth, the nozzle 20 may be inserted into the pore, and the sterilization composition 10 may be allowed to flow into the pore to achieve efficient sterilization.

When the object 6 is not a tubular body (for example, forceps or an implant), sterilization can be performed by, for example, spraying the sterilization composition 10 onto the object 6 with the nozzle 20. In the case where the object 6 is located inside the body (for example, an implant), it is preferable that the sterilization system 100 includes a discharge part for suctioning and discharging the used sterilization composition 10 after it has been sprayed onto the object 6.

The biofilm 2 is a higher-order structure formed by bacteria 1 adhering to the surface of a solid phase (object 6) and is, for example, covered with polysaccharides produced by the bacteria 1. The bacteria 1 contained in the biofilm 2, that is, the bacteria 1 serving as the sterilization target in the present embodiment, are not particularly limited and may be either Gram-positive or Gram-negative bacteria. For example, the sterilization method of the present embodiment is also effective against Klebsiella pneumoniae (Gram-negative), Pseudomonas aeruginosa (Gram-negative), and Staphylococcus epidermidis (Gram-positive), which are specified by the U.S. Food and Drug Administration (FDA) as bacteria that significantly affect endoscope contamination.

In addition, when the bacteria contained in the biofilm are Gram-negative bacilli, particularly Gram-negative bacilli belonging to the family Enterobacteriaceae, the electron transfer (arrow 4 in Figs. 2 and 3) becomes more efficient, and a more excellent sterilization effect can be obtained. Examples of bacteria belonging to the family Enterobacteriaceae include, for example, the above-mentioned Klebsiella species, Enterobacter species, Escherichia species, Salmonella species, Serratia species, Shigella species, and Yersinia species.

### [Sterilization Method]

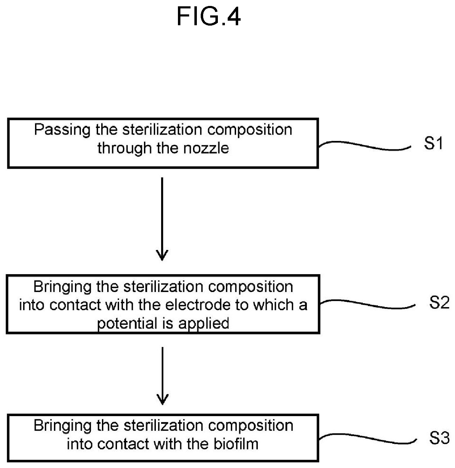

A method for sterilizing a biofilm using the sterilization system 100 shown in Fig. 1 will be described. The sterilization method of the present embodiment includes, for example, the following steps (see Fig. 4):
Step S1: Passing the sterilization composition 10 through the nozzle 20 (electrode 40).
Step S2: Bringing the sterilization composition 10 into contact with the electrode 40 to which a potential exceeding the lower limit of the potential window and being -0.4 V or lower with reference to a silver/silver chloride electrode is applied.
Step S3: Bringing the sterilization composition 10, which has passed through the nozzle 20 (that is, has contacted the electrode 40), into contact with the biofilm 2.

### Step S1: Passing the sterilization composition 10 through the nozzle 20

The sterilization composition 10 is passed through the nozzle 20 by, for example, using a liquid feeding mechanism (not shown). The speed (feeding rate) of the sterilization composition 10 passing through the nozzle 20 is not particularly limited, and may be, for example, 0.1 mL/min to 10 mL/min.

### Step S2: Bringing the sterilization composition 10 into contact with the electrode 40 to which a potential is applied

The application of the potential to the electrode 40 is performed by the control part 70. In the sterilization system 100 of the present embodiment, it is preferable to use the three-electrode method, and the control part 70 is preferably a potentiostat. The magnitude of the applied potential is as described above. The potential may be applied continuously or intermittently, but from the viewpoint of efficient sterilization, it is preferable to apply it continuously. The specific organic compound 5 contained in the sterilization composition 10 receives electrons from the electrode 40 while passing through the nozzle 20, thereby increasing its reducing ability.

In the embodiment shown in Fig. 1 (an embodiment in which the nozzle 20 also functions as the electrode 40), the sterilization composition 10 passes through the nozzle 20 while coming into contact with the electrode 40 to which a potential is applied. That is, Steps S1 and S2 are carried out in parallel along the time axis. On the other hand, in an embodiment in which, for example, an electrode 40 separate from the nozzle 20 is provided at the tip of the nozzle 20, Step S2 is carried out after Step S1. As understood from the above description, the temporal order of Steps S1 and S2 is not particularly limited.

### Step S3: Bringing the sterilization composition 10 into contact with the biofilm 2

The method for bringing the sterilization composition 10 that has come into contact with the electrode 40 (that is, the sterilization composition 10 containing the specific organic compound 5 whose reducing ability has been enhanced) into contact with the biofilm 2 is not particularly limited. For example, when sterilizing a biofilm formed on the inner surface of a tubular body, as shown in Figs. 1 and 2, the nozzle 20 and the object (tubular body) 6 may be connected, and the sterilization composition 10 may be allowed to flow from the nozzle 20 into the interior of the object (tubular body) 6. In another example, when sterilizing a biofilm formed inside pores, the nozzle 20 may be inserted into the pores and the sterilization composition 10 may be allowed to flow into the pores. The sterilization composition 10 may also be discharged from the nozzle 20 toward the biofilm. The sterilization composition 10 that has passed through the nozzle 20 contains the specific organic compound 5 whose reducing ability has been enhanced. By bringing this composition into contact with or close to the biofilm 2, the bacteria 1 within the biofilm 2 can be sterilized.

The time during which the sterilization composition 10 is brought into contact with the biofilm 2 (sterilization time) is not particularly limited and may be appropriately adjusted according to factors such as the type of specific organic compound, the type of bacteria, and the liquid feeding rate. For example, the sterilization time may be 30 minutes to 24 hours. The temperature (sterilization temperature) of the sterilization composition 10 brought into contact with the biofilm 2 is also not particularly limited. For example, the sterilization temperature may be 0°C to 100°C, or room temperature.

The contact of the sterilization composition 10 with the biofilm 2 may be carried out under anaerobic conditions without oxygen (for example, in a nitrogen atmosphere), or under aerobic conditions containing oxygen (for example, in the atmosphere). Since the atmosphere is not limited to anaerobic conditions, the sterilization method of the present embodiment can be carried out with simpler equipment.

According to the sterilization system 100 of the present embodiment and the sterilization method using the sterilization system 100 described above, it is possible to reduce the activity of the bacteria 1 in the biofilm 2 and ultimately kill (sterilize) all or part of the bacteria in the biofilm 2 simply by applying a weak potential to the electrode 40. Therefore, a continuous effect against drug-resistant bacteria residing in the biofilm 2 can be expected. In addition, in the sterilization system 100, by providing the electrode 40 in the nozzle 20, it becomes easier to deliver the specific organic compound 5, whose reducing ability has been enhanced, to the biofilm 2, thereby further enhancing the sterilization effect. By using the sterilization system 100 of the present embodiment, the use of antibacterial agents can be eliminated or reduced, and the occurrence of new drug-resistant bacteria can also be suppressed.

### [Example]

The present invention will be described in more detail below based on Examples. The materials, amounts used, ratios, treatment contents, treatment procedures, and the like described in the following Examples can be appropriately modified as long as they do not depart from the spirit of the present invention. Therefore, the scope of the present invention should not be interpreted as being limited by the following Examples.

### [Experiment 1]

In this experiment, the sterilization of Pseudomonas aeruginosa (PA) present in a biofilm formed inside a PTFE tube (object 6) was performed using the sterilization system 100 shown in Fig. 1. In this experiment, a three-electrode method was employed in which the electrode (working electrode) 40, the counter electrode 50, and the reference electrode 60 were controlled by a potentiostat (control part 70). The nozzle 20 was entirely constituted by the electrode 40 (SUS tube, diameter: 0.41 cm, length: 1 cm). As the specific organic compound, methyl viologen (MV) was used.

### (1) Formation of Biofilm

First, 15 mL of LB (Luria-Bertani) medium was prepared in a 50 mL tube, and a frozen PA bacterial sample (-80°C) was added thereto. The sample was shaken and incubated overnight at 37°C, and then further cultured under aerobic conditions. Next, a PTFE tube (diameter: 0.5 cm, length: 20 cm) was sterilized in an autoclave at 120°C for 20 minutes. The sterilized PTFE tube was filled with TSB (Tryptic Soy Broth) medium containing the cultured PA bacteria (OD₆₀₀ = 0.01), both ends of the tube were sealed with stoppers, and the tube was left to stand in an incubator at 37°C for 24 hours. As a result, a biofilm was formed on the inner surface (inner wall) of the PTFE tube.

### (2) Preparation of Sterilization Composition

Methyl viologen (MV) was added to a defined medium (DM; liquid medium) to a final concentration of 500 µM and mixed uniformly. The DM medium used was an electrolyte solution containing the following electrolytes: CaCl₂·2H₂O: 0.08 g/L, NH₄Cl: 1.0 g/L, NaHCO₃: 2.5 g/L, NaCl: 10.0 g/L, MgCl₂·6H₂O: 0.2 g/L, and HEPES: 7.2 g/L.

### (3) Sterilization of Biofilm

A platinum (Pt) wire was used as the counter electrode 50, and a silver/silver chloride electrode was used as the reference electrode 60. These electrodes were electrically connected to the potentiostat (control part 70) together with the working electrode 40 (nozzle 20). The prepared sterilization composition 10 was poured into the sterilization composition supply part 30 (glass beaker), and the counter electrode 50 and the reference electrode 60 were placed in contact with the sterilization composition 10. The sterilization composition supply part 30 and the nozzle 20 (SUS tube) were connected using a PTFE tube 32 and a joint 33 (silicone tube). The nozzle 20 (SUS tube) and one end of the PTFE tube (object 6) on which the biofilm 2 had been formed were connected using a joint 21 (silicone tube). Furthermore, a drain pipe (not shown) was connected to the other end of the PTFE tube (object 6). As a liquid feeding device, a syringe pump (not shown, inlet pump) was connected to the sterilization composition supply part 30, and a tube roller pump (not shown, outlet pump) was connected to the drain pipe.

The liquid feeding devices (syringe pump and tube pump) were operated to flow the sterilization composition 10 sequentially from the sterilization composition supply part 30 through the nozzle 20, the PTFE tube (object 6), and the drain pipe (not shown). The liquid feeding rate was set at 2 mL/min. A potential of -1.0 V (vs Ag/AgCl) was applied to the working electrode 40. Under the applied potential, the sterilization composition was circulated for one hour. This experiment was conducted under aerobic conditions (in the atmosphere).

### [Evaluation of Sterilization Effect]

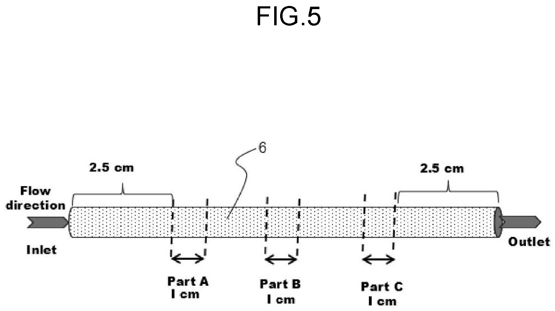

From the PTFE tube (object 6) after the experiment, three sections, A (1 cm), B (1 cm), and C (1 cm), as shown in Fig. 5, were cut out. Section A corresponds to the portion 2.5 cm to 3.5 cm from the upstream end (one end) of the PTFE tube, section C corresponds to the portion 2.5 cm to 3.5 cm from the downstream end (the other end) of the PTFE tube, and section B corresponds to the substantially central portion of the PTFE tube.

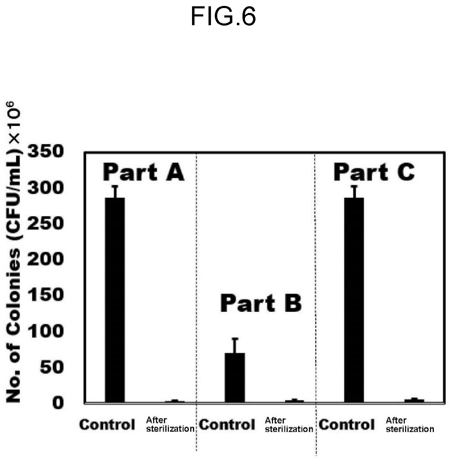

Each of the sections A, B, and C was subjected to the following procedures. First, each section was washed three times with PBS (pH = 7.2). Each washed section was placed in a container, and 1 mL of PBS was added thereto. Strong vortex mixing (for 30 seconds), ultrasonic treatment (for 7 minutes), and further strong vortex mixing (for 30 seconds) were performed to detach the biofilm from each section. The PBS containing the detached biofilm was diluted by a factor of 10⁷. A 10 µL portion of the diluted PBS containing the biofilm was spread on a TSB-agar medium plate to prepare samples, which were then incubated overnight at 37°C. After incubation, the number of colonies formed on each sample was counted, and the colony count (colony-forming units (CFU/mL)) was determined. The same experiment was repeated three times, and the average values were obtained. The results are shown in Fig. 6. For comparison, as a control, the same procedure was performed using a PTFE tube in which the biofilm 2 had been formed but no sterilization treatment was performed thereafter, and the colony counts of the three sections (A, B, and C) were similarly determined. These results are also shown in Fig. 6.

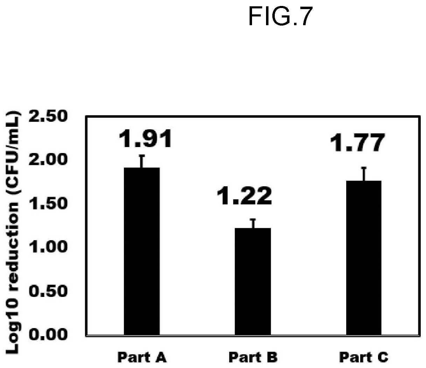

Furthermore, to clarify the experimental results, Fig. 7 shows the bacterial reduction amounts (log₁₀ bacterial reduction) for each of sections A, B, and C. The bacterial reduction amount represents the difference obtained by subtracting the colony count (log₁₀) of each section after sterilization from the colony count (log₁₀) of the corresponding control (before sterilization) shown in Fig. 6.

As shown in Figs. 6 and 7, in all of the sections A, B, and C, the number of bacterial colonies after sterilization decreased compared to the control (before sterilization), confirming the effectiveness of the sterilization method using the sterilization system 100.

In Fig. 7, the bacterial reduction in section B (the central part of the PTFE tube) is slightly lower than in sections A and C (near both ends of the PTFE tube). This is attributed to the fact that biofilm formation is less likely to occur in the central part of the PTFE tube (object 6). Therefore, this result does not indicate that there is variation in the sterilization effect depending on the position in the PTFE tube. On the other hand, as shown in Fig. 7, similar sterilization effects were obtained at both ends of the PTFE tube (section A near the upstream end and section C near the downstream end). This demonstrates that the same effect can be obtained even at distant positions, indicating the practicality of the present invention.

### [Experiment 2]

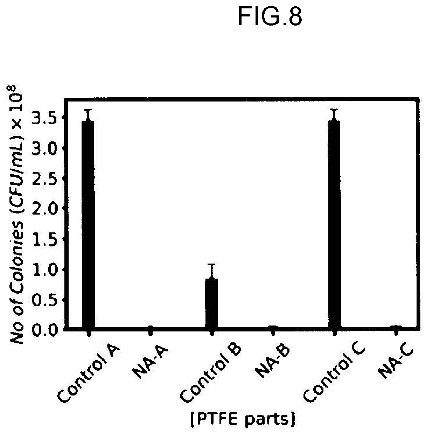

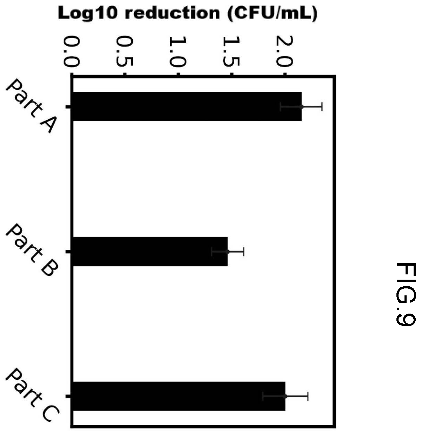

A sterilization experiment was conducted under the same conditions as in Experiment 1, except that the length of the electrode 40 (nozzle 20, SUS tube) was set to 5 cm, and the evaluation was performed in the same manner. The results are shown in Figs. 8 and 9. As shown in Figs. 8 and 9, sterilization effects were also obtained in Experiment 2, similarly to Experiment 1.

### [Calculation of Current Density]

The current values for the 5 cm SUS tube and the 10 cm SUS tube used as electrodes in Experiment 2 were measured, and the current density was calculated from the results according to the following procedure.

First, the current value flowing through the 5 cm SUS tube was measured using the potentiostat (control part 70). In addition, a sterilization experiment was conducted under the same conditions as in Experiment 2, except that the length of the electrode 40 (nozzle 20, SUS tube) was set to 10 cm, and the current value for the 10 cm SUS tube was similarly measured. Since the magnitude of the background current specific to the system does not depend on the size of the electrode, it was approximately the same for the 5 cm and 10 cm SUS tubes.

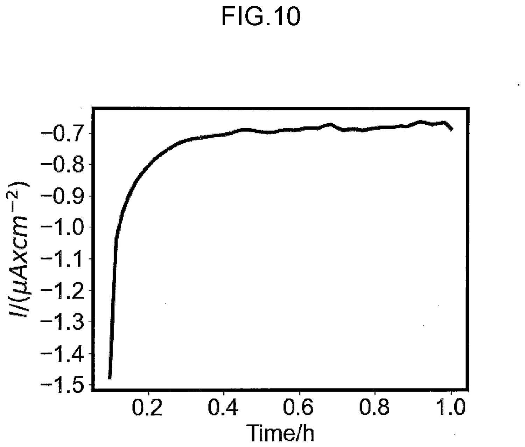

On the other hand, the magnitude of the current involved in the sterilization reaction depends on the size of the electrode (that is, the length of the SUS tube). Therefore, by subtracting the current value of the 5 cm SUS tube from that of the 10 cm SUS tube (length difference: 5 cm), the current value involved in the sterilization reaction flowing through the 5 cm SUS tube was obtained. This value was divided by the inner surface area of the 5 cm SUS tube (the contact area between the electrode and the sterilization composition) to calculate the current density. The results are shown in Fig. 10. As shown in Fig. 10, the current density was approximately -0.7 µA/cm².

### [Industrial Applicability]

The sterilization method of the present invention can be utilized for cleaning and maintenance to prevent infections caused by medical instruments and medical implants.

### [Explanation of Reference Numerals]

- 1: Bacterial cell (bacterium)
- 2: Biofilm
- 5: Specific organic compound
- 6: Object
- 10: Sterilization composition
- 20: Nozzle
- 21: Joint
- 30: Sterilization composition supply part
- 31: Container
- 32: Tube
- 33: Joint
- 40: Electrode (working electrode)
- 50: Counter electrode
- 60: Reference electrode
- 70: Control part
- 71: Wiring
- 100: Sterilization system

## Claims

1. A sterilization system for sterilizing a biofilm formed on an object, comprising:
a sterilization composition containing an organic compound having a standard redox potential (pH 7) of -0.7 V to -0.2 V and water;
a sterilization composition supply part that holds the sterilization composition;
a nozzle that supplies the sterilization composition held in the sterilization composition supply part to the biofilm;
an electrode provided in the nozzle so as to be in contact with the sterilization composition; and
a control part connected to the electrode and configured to apply to the electrode a potential exceeding the lower limit of a potential window and being -0.4 V or lower with reference to a silver/silver chloride electrode.

2. The sterilization system according to claim 1, wherein the electrode is constituted by at least a part of the nozzle.

3. The sterilization system according to claim 1, wherein the electrode is constituted by the entire nozzle.

4. The sterilization system according to any one of claims 1 to 3, wherein the electrode has a ring shape.

5. The sterilization system according to any one of claims 1 to 4, wherein the electrode is a working electrode, and further comprises a counter electrode and a reference electrode that are provided so as to be in contact with the sterilization composition.

6. The sterilization system according to claim 5, wherein the counter electrode and the reference electrode are provided in the sterilization composition supply part.

7. The sterilization system according to any one of claims 1 to 6, wherein the organic compound contained in the sterilization composition is methyl viologen.

8. The sterilization system according to any one of claims 1 to 7, wherein the sterilization composition further contains cations.

9. The sterilization system according to any one of claims 1 to 8, wherein the sterilization composition further contains silver ions.

10. The sterilization system according to any one of claims 1 to 9, wherein the object is a medical instrument.

11. The sterilization system according to any one of claims 1 to 10, wherein the object is a tubular body, and the biofilm is formed on an inner surface of the tubular body.

12. The sterilization system according to any one of claims 1 to 11, wherein the object is an insertion tube of an endoscope.

13. The sterilization system according to any one of claims 1 to 12, wherein the control part applies a potential of -1.2 V to -0.8 V to the electrode.

14. A method for sterilizing a biofilm formed on an object using the sterilization system according to any one of claims 1 to 13, comprising:
bringing the sterilization composition into contact with the electrode to which a potential exceeding the lower limit of a potential window and being -0.4 V or lower with reference to a silver/silver chloride electrode is applied; and
bringing the sterilization composition that has contacted the electrode into contact with the biofilm.
